# EUROPÄISCHE PATENTANMELDUNG

(11) **EP 2 801 319 A1**
(43) Veröffentlichungstag der Anmeldung: **12.11.2014**
(21) Anmeldenummer: 13002407.8
(22) Anmeldetag: 06.05.2013
(51) Int. Cl.: A61B 6/04, A61B 5/11

(54) **Vorrichtung zur Lagerung der unteren Extremitäten eines Patienten während der radiologischen Diagnostik bzw. Behandlung**

(71) Anmelder: IT-V Medizintechnik GmbH, 6020 Innsbruck (AT)
(72) Erfinder: Völp, Markus, 6020 Innsbruck (AT)
(74) Vertreter: Herrmann, Uwe

(57) **Zusammenfassung**

Die Erfindung betrifft eine Vorrichtung zur Lagerung der unteren Extremitäten eines Patienten während der radiologischen Diagnostik bzw. Behandlung, wobei die Vorrichtung wenigstens ein Fußteil aufweist, das wenigstens eine rotierbar gelagerte Halterung für einen einzelnen Fuß und/oder den Unterschenkel des Patienten aufweist. Die Erfindung betrifft ferner eine gattungsgemäße Vorrichtung, wobei das Fußteil eine Halterung für einen einzelnen Fuß und/oder den Unterschenkel des Patienten aufweist, und wobei die Halterung Mittel zur festen Fixierung bzw. Immo-bilisierung des einzelnen Fußes und/oder Unter-schenkels des Patienten aufweist.

## Beschreibung

Die vorliegende Erfindung betrifft eine Vorrichtung zur Lagerung der unteren Extremitäten eines Patienten während der radiologischen Diagnostik bzw. Behandlung.

Gattungsgemäße Vorrichtungen sind aus dem Stand der Technik bekannt und dienen dazu, die unteren Extremitäten des Patienten während einer radiologischen Behandlung wie beispielsweise einer Bestrahlung zu fixieren. Die Anmelderin vertreibt unter der Handelsbezeichnung ProSTEP eine solche Vorrichtung, wobei der Knie- bzw. Oberschenkelbereich beider Beine auf einem Beinteil gelagert ist, und wobei die Füße des Patienten auf einem in Längsrichtung der Vorrichtung verschiebbaren Fußteil gelagert sind. Die Macromedics BV vertreibt unter der Handelsbezeichnung LEX-ULTRA eine weitere gattungsgemäße Vorrichtung, wobei die Beine individuell voneinander höhenverstellbar gelagert und mit einem thermoplastischen Maskenmaterial auf einer Unterlage fixiert werden.

Bei manchen Behandlungssituationen wie beispielsweise bei der Bestrahlung von Sarkomen kann es wünschenswert sein, einzelne Beine individuell voneinander zu positionieren und insbesondere auch eine bestimmte Rotationsstellung eines einzelnen Beines auszuwählen und zu fixieren, beispielsweise durch Ausdrehen des Fußes. Gleichzeitig soll die Handhabung der Vorrichtung möglichst einfach gestaltet sein und das Bein sicher und auch komfortabel für den Patienten fixiert werden. Unabhängig von der Rotationsstellung ist auch ganz allgemein eine Immobilisierung des Fußes des Patienten erstrebenswert, welche mit bekannten Vorrichtungen nur unzureichend erreicht wird oder einen komplizierten Arretierungsvorgang durch den Benutzer erfordert.

Ziel der Erfindung ist es, eine gattungsgemäße Vorrichtung bereitzustellen, die diesen Anforderungen genügt.

Vor diesem Hintergrund ist erfindungsgemäß eine Vorrichtung zur Lagerung der unteren Extremitäten eines Patienten während der radiologischen Diagnostik bzw. Behandlung vorgesehen, wobei die Vorrichtung wenigstens ein Fußteil aufweist, das seinerseits wenigstens eine rotierbar gelagerte Halterung für einen einzelnen Fuß und/oder den Unterschenkel des Patienten aufweist. Dadurch wird erreicht, dass ein Fuß bzw. Bein unabhängig vom anderen gedreht und in einer bestimmten Rotationsstellung fixiert werden kann. Die Rotationsachse wird so gewählt, dass ein Ausdrehen bzw. Eindrehen des Fußes erfolgen kann und verläuft daher im Wesentlichen parallel zum Unterschenkel des in der Halterung gelagerten Beines. Das bedeutet, dass die Rotationsachse im Wesentlichen in Längsrichtung der Vorrichtung verläuft und gegebenenfalls in vertikale Richtung aufgeklappt werden kann. Zum Zweck der Rotation weist das Fußteil ein Rotationslager auf, mit dem die Halterung unmittelbar oder mittelbar verbunden ist.

Ferner ist vor dem oben genannten Hintergrund eine gattungsgemäße Vorrichtung vorgesehen, wobei das Fußteil eine Halterung für einen einzelnen Fuß und/oder den Unterschenkel des Patienten aufweist. Erfindungsgemäß weist die Halterung Mittel zur festen Fixierung bzw. Immobilisierung des einzelnen Fußes und/oder Unterschenkels des Patienten auf.

In einer Ausführungsform weist die Halterung eine Fußauflage und eine Befestigungsvorrichtung für den Fuß auf, die ein derartiges Mittel zur festen Fixierung bzw. Immobilisierung des einzelnen Fußes und/oder Unterschenkels des Patienten darstellt. Vorzugsweise ist vorgesehen, dass es sich bei der Halterung um eine Bindung handelt. Die Fußauflage kann beispielsweise als Platte, Schale oder Matte zum Auflegen der Fußsohle ausgebildet sein oder ein derartiges Bauteil aufweisen. Die Befestigungsvorrichtung kann einen Schaft für den Unterschenkel und/oder ein Mittel zur Verhinderung eines Abhebens des Fußes aufweisen. Geeignete derartige Mittel umfassen Ratschenbänder, Schnürsenkel, elastische Riemen, Schlaufen oder Hülse. Der Schaft bzw. die Mittel können integral mit der Fußauflage gefertigt sein oder von dieser abnehmbar sein. In einer bevorzugten Ausgestaltung wird als Bindung eine Snowboardbindung verwendet, wobei eine Platte als Fußauflage dient und ein Schaft und Ratschenbänder zur Fixierung des Fußes und unteren Beinbereichs dienen.

In einer Ausführungsform weist das Fußteil eine Basis auf und die Halterung bzw. Bindung ist rotierbar an der Basis gelagert. Bei der Basis handelt es sich beispielsweise um einen im Wesentlichen in Längsrichtung der Vorrichtung bzw. der Beine des Patienten verschiebbaren Schlitten. Durch diese verschiebbare Anordnung kann eine Anpassung auf verschiedene Beinlängen erfolgen und es können verschiedene Winkelstellungen des Beines eingestellt werden. Durch die Konstruktion einer verschiebbaren Basis, auf der die Halterung rotierbar angelenkt ist, erfolgt eine Entkoppelung der Schiebebewegung und der Rotationsbewegung, was eine einfachere und sicherere Handhabung durch den Benutzer ermöglicht und gleichzeitig eine erhöhte Stabilität der Vorrichtung mit sich bringt.

Alternativ ist auch eine andere Ausbildung des Fußteils denkbar, wie beispielsweise eine höhenverstellbare Auflage für Teilbereiche oder der ganzen Teil eines Beines, auf dem die Halterung rotierbar gelagert sind.

In einer Ausführungsform ist die Halterung bzw. Bindung abnehmbar an der Vorrichtung bzw. der Basis gelagert.

In einer Ausführungsform ist die Halterung bzw. Bindung ferner um eine im Wesentlichen in Querrichtung der Vorrichtung bzw. der Beine des Patienten verlaufende Achse schwenkbar gelagert. Durch diese schwenkbare Lagerung kann der Winkel des Kniegelenks und/oder des Sprunggelenks des Patienten gewählt und fixiert werden, beispielsweise im Zusammenwirken mit einer in Längsrichtung der Vorrichtung verschiebbaren Ausführung der Basis und/oder mit einem höhenverstellbaren Beinteil, welches das Bein im Kniebereich bzw. Oberschenkelbereich stützt. Vorzugsweise ist die Halterung bzw. Bindung schwenkbar an der Basis oder an dem Schlitten gelagert. Zum Zweck der Schwenkbewegung weist das Fußteil ein Schwenklager auf, mit dem die Halterung bzw. Bindung unmittelbar oder mittelbar verbunden ist. Bei dem Schwenklager und dem Rotationslager kann es sich um ein gemeinsames Gelenk oder zwei individuelle Gelenke handeln. Letzteres kann aus Gründen der Handhabbarkeit und Stabilität bevorzugt sein. Im Falle zweier individueller Gelenke kann zwischen dem Rotationslager und dem Schwenklager ein Zwischenstück angeordnet sein. Denkbar ist es, dass an der Basis bzw. dem verschiebbaren Schlitten unmittelbar das Schwenklager angeordnet ist, an dem ein Zwischenstück mit dem Rotationslager anschließt. An dem Rotationslager ist letztlich die Halterung bzw. Bindung angeordnet.

In einer Ausführungsform weist das Fußteil zwei unabhängig voneinander rotierbar und/oder schwenkbar an der Basis gelagerte Halterungen bzw. Bindungen für die beiden Füße des Patienten auf. So ist es möglich, einen gemeinsamen Schlitten und gegebenenfalls ein gemeinsames Schwenklager für beide Beine zu verwenden und dennoch die Rotationsstellung beider Beine bzw. Füße individuell wählen zu können. Dies kann aus Gründen der Handhabbarkeit und Stabilität bevorzugt sein. Die einzelnen Halterungen bzw. Bindungen sind vorzugsweise baugleich und/oder spiegelverkehrt zueinander auf der Basis angeordnet. Alternativ ist es denkbar, an der Vorrichtung zwei oben näher beschriebene Fußteile vorzusehen. Diese sind vorzugsweise baugleich und/oder spiegelverkehrt zueinander. Im Falle von zwei Fußteilen kann ein oder können beide Fußteile von der Vorrichtung bzw. der Platte abnehmbar sein.

In einer Ausführungsform weist die Vorrichtung ferner ein Beinteil mit einer Beinauflage für ein einzelnes Bein auf, wobei die Beinauflage auf einem höhenverstellbaren Träger gelagert ist. Die Beinauflage dient zur Lagerung des Beins im Bereich des Knies und/oder des Oberschenkels. Durch eine derartige Ausgestaltung kann ein Bein unabhängig vom anderen Bein gehoben und gesenkt werden. Im Zusammenspiel mit dem individuell rotierbaren und gegebenenfalls ferner längsverschiebbaren und/oder schwenkbaren Fußteil kann so eine maximale Flexibilität in der Arretierposition für jedes einzelne Bein und jeden einzelnen Fuß erreicht werden. Das höhenverstellbare Beinteil kann auch verwendet werden, um ein Bein abzuheben und so eine Bestrahlungsbahn für die Innenseite des anderen Beines freizugeben.

In einer Ausführungsform weist die Vorrichtung eine Grundplatte auf, auf der das Fußteil und/oder das Beinteil angeordnet sind. Beispielsweise ist die Basis des Fußteils bzw. der Schlitten in Längsrichtung der Vorrichtung verschiebbar auf der Grundplatte gelagert. Zu diesem Zweck kann die Grundplatte eine Schiene oder Führung für die Basis bzw. den Schlitten aufweisen. Eine Grundplatte hat sich als geeigneter Grundkörper für eine gattungsgemäße Vorrichtung bewährt und kann auf eine Vielzahl von radiologischen Behandlungsvorrichtungen aufgesetzt werden. Das Beinteil kann, sofern vorhanden, auf derselben Grundplatte wie das Fußteil angeordnet sein. Dadurch wird die Position des Beinteils und des Fußteils zueinander definiert und die gesamte Vorrichtung kann in einfacher Weise bedient und am radiologischen Behandlungsplatz integriert werden.

In einer Ausführungsform handelt es sich bei dem Träger um einen gegebenenfalls senkrecht auf die Translationsebene des Schlittens bzw. auf die Grundplatte stehenden Mast. Dieser kann in seiner Länge verstellbar sein, um die Höhe der Beinauflage auf der Vorrichtung bzw. im Verhältnis zur Grundplatte verändern zu können. Beispielsweise kann vorgesehen sein, dass der Mast teleskopartig aus- und einfahrbar ist. In diesem Zusammenhang kann eine zweigliedrige Ausführung vorgesehen sein, um die Handhabung so einfach wie möglich zu halten.

In einer Ausführungsform weist die Beinauflage eine Matte auf, die vorzugsweise in einem Rahmen oder auf einer Auflage gehaltert ist. Gegebenenfalls kann die Beinauflage eine Ausformung für das Bein aufweisen.

In einer Ausführungsform ist das Beinteil abnehmbar an der Vorrichtung oder der Grundplatte gelagert. So kann erreicht werden, dass ein Bein fixiert werden kann, während das andere Bein abgespreizt oder bequem angelegt werden kann. So wird die Flexibilität der Behandlung weiter erhöht und die unteren Extremitäten des Patienten werden nur in dem benötigten Ausmaß fixiert.

In einer Ausführungsform weist die Vorrichtung eine Arretiervorrichtung und vorzugsweise eine Rastvorrichtung auf, die so ausgebildet ist, dass die Rotationsstellung der Halterung bzw. Bindung stufenlos oder in Stufen arretierbar ist. Gleichsam kann die Vorrichtung Arretiervorrichtungen und vorzugsweise Rastvorrichtungen aufweisen, die so ausgebildet sind, dass die Translationsstellung des Schlittens und/oder die Schwenkstellung der Halterung bzw. Bindung und/oder die Höhe des Trägers stufenlos oder in Stufen arretierbar sind.

Weitere Einzelheiten und Vorteile der Erfindung ergeben sich aus dem nachfolgend anhand der Figuren diskutierten Ausführungsbeispiel. In den Figuren zeigen:
- Figur 1:: eine erste Ansicht einer Ausführungsform einer erfindungsgemäßen Vorrichtung,
- Figur 2:: eine weitere Ansicht dieser Vorrichtung,
- Figur 3:: eine Detailansicht des Fußteils dieser Vorrichtung,
- Figur 4:: eine weitere Detailansicht des Fußteils dieser Vorrichtung, und
- Figur 5:: eine Darstellung dieser Vorrichtung, wobei die unteren Extremitäten eines Patienten daran fixiert sind.

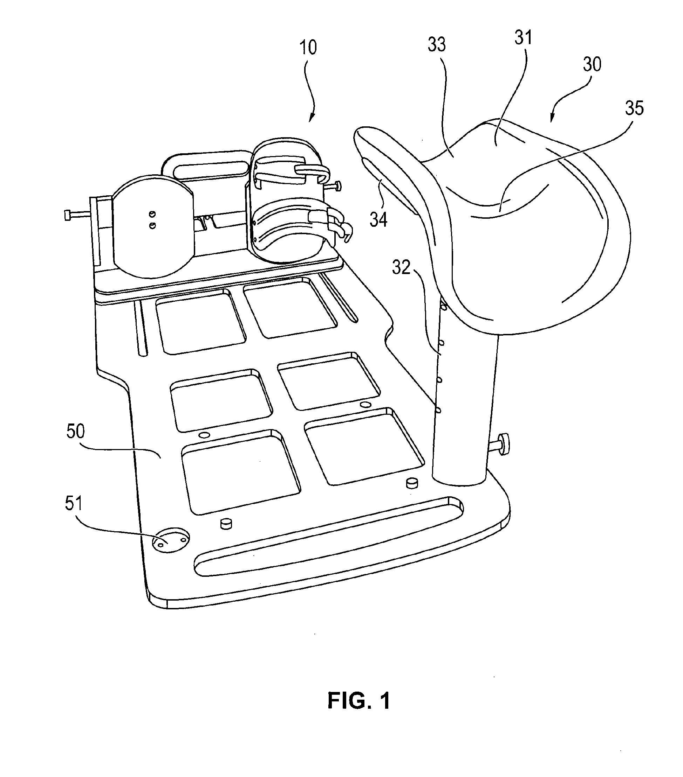

In Figur 1 wird von schräg hinten eine Ausführungsform einer erfindungsgemäßen Vorrichtung gezeigt, wobei ein Fußteil 10 und ein Beinteil 30 auf einer gemeinsamen Grundplatte 50 angeordnet sind. Die Grundplatte 50 kann aus faserverstärktem Kunststoff, Metall oder anderen geeigneten Materialen gefertigt sein.

Das Beinteil 30 weist eine Beinauflage 31 und einen Träger 32 auf. Es ist in der gezeigten Ausführungsform so an der Grundplatte 50 fixiert, dass es zur Auflage des rechten Beines eines Patienten dient. Der Träger 32 ist abnehmbar an der Grundplatte 50 gelagert und kann alternativ auch in einer Halterung 51 an der linken Seite der Grundplatte 50 eingesetzt werden, um eine Auflage des linken Beines des Patienten zu gewährleisten. Die Vorrichtung kann alternativ auch mit zwei abnehmbar an der Grundplatte 50 gelagerten Beinteilen 30 ausgestattet sein, um beide Beine höhenverstellbar lagern zu können. Sollte eine derartige Lagerung der Beine nicht erwünscht sein, kann das Beinteil 30 bzw. können auch beide Beinteile abgenommen werden.

Die Beinauflage 31 dient zur Stützung des Kniebereichs und des unteren Teils des Oberschenkels. Sie weist eine ausgeformte Matte 33 auf, die auf einer Auflage 34 gehaltert ist. Die Ausformung ist an der Form des gelagerten Beines orientiert und weist ein Tal mit einem Knick 35 auf, der sich im Bereich der Kniekehle befindet. Die Matte 33 kann beispielsweise aus Schaumstoff gefertigt sein und einen Überzug aus Kunststoff aufweisen.

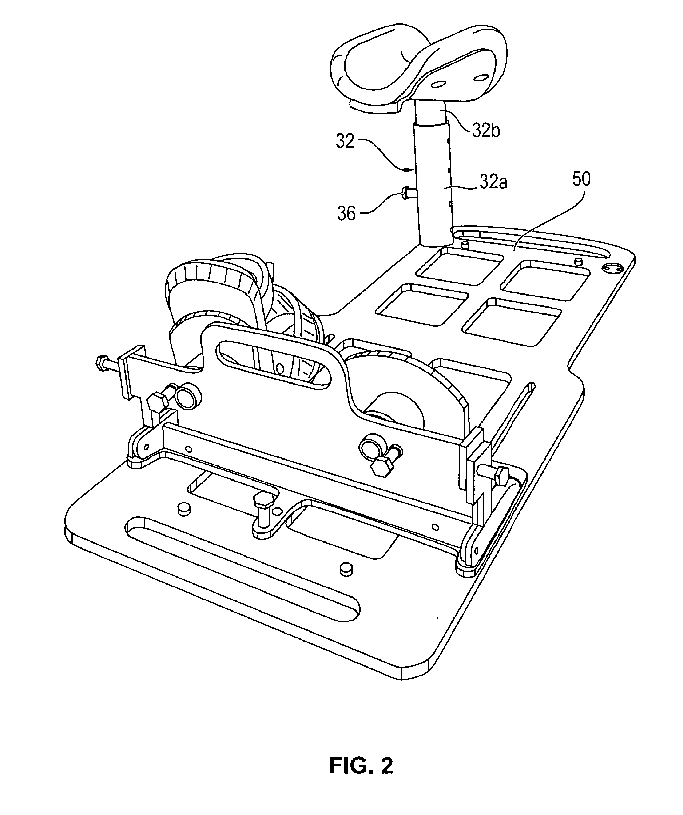

Der Träger 32 kann mit Blick auf Figur 2 besser erkannt werden und ist als Mast ausgebildet, der zwei teleskopartig aus- und einfahrbare Glieder 32a und 32b aufweist. Selbstverständlich ist auch eine mehrgliedrige Ausführung denkbar. Das obere Mastglied 32b ist mit der Auflage 34 verbunden und das untere Mastglied 32a ist lösbar mit der Grundplatte 35 verbunden. Der Mast 32 erstreckt sich in der gezeigten Ausführungsform senkrecht von der Grundplatte 50 nach oben. Selbstverständlich ist auch eine gewinkelte Anordnung denkbar. Die teleskopartige Verstellbarkeit der Mastglieder 32a und 32b zueinander führt zu einer Höhenverstellbarkeit der Beinauflage 31 gegenüber der Grundplatte 50. Die Mastglieder 32a und 32b können zueinander in mehreren Stufen fixiert werden. Beispielsweise kann am unteren Mastglied 32a ein vorgespannter Stift 36 angeordnet sein, der wahlweise in eines von mehreren in Längsrichtung des oberen Mastglieds 32b beabstandeten Löchern eingreift. Selbstverständlich sind auch andere Mittel denkbar, mit denen eine stufenlose oder stufige Arretierung der Mastglieder zueinander erreicht werden kann.

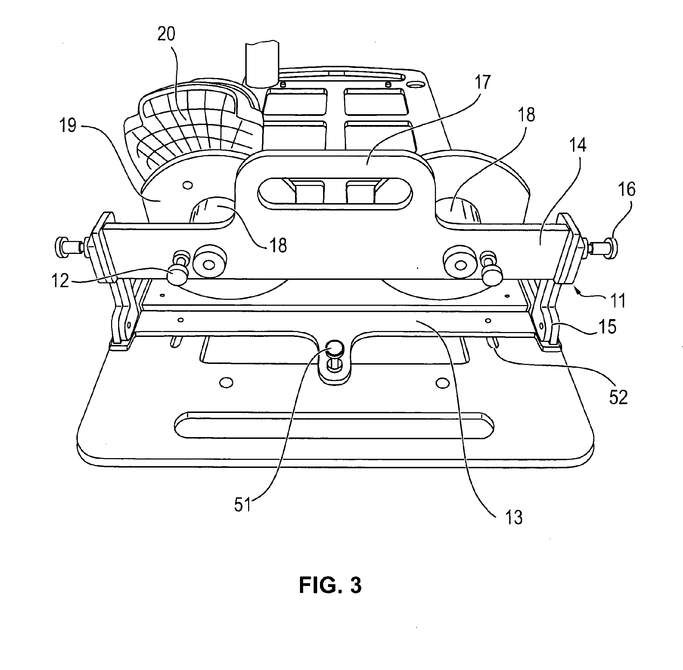

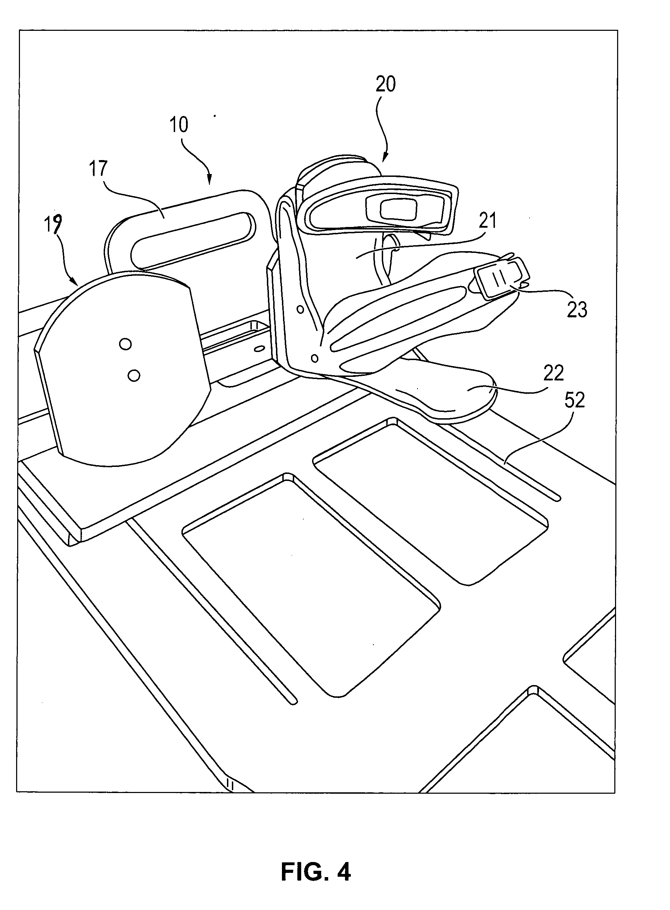

Die Ausgestaltung des an der Vorderseite der Grundplatte 50 angeordneten Fußteils 10 wird im Folgenden mit Blick auf die Figuren 3 und 4 erläutert.

Das Fußteil weist eine Halterung bzw. Bindung 20 auf, die drehbar an einer Basis 11 angeordnet ist. Bei der Halterung bzw. Bindung handelt es sich im gezeigten Ausführungsbeispiel um eine modifizierte Snowboardbindung mit einem plattenförmigen Fußbett 21, einem ausgepolsterten Schaft 22 für den Unterschenkel und einer zweiteiligen Ratschenverbindung 23. Selbstverständlich sind auch andere Bindungsformen denkbar, die eine Arretierung des Fußbereiches ermöglichen. Die Bindung 20 ist drehfest an einer Drehscheibe 19 befestigt, die über ein Drehgelenk 18 rotierbar an einer Basis 11 angelenkt ist. Die Drehachse verläuft in der gezeigten Grundstellung in etwa in Längsrichtung der Vorrichtung. Durch ein später näher erläutertes Schwenken der Bindung kann diese aber auch nach oben geneigt werden. Die Rotationsstellung der Bindung kann in mehreren Stufen fixiert werden. Vorliegend ist an der Basis 11 ein vorgespannter Stift 12 angeordnet, der wahlweise in eines von mehreren in Umfangsrichtung der Drehscheibe 19 bzw. des Drehgelenks 18 beabstandeten Löchern eingreift. Selbstverständlich sind auch andere Mittel denkbar, mit denen eine stufenlose oder stufige Arretierung der Rotationsstellung erreicht werden kann.

Die Basis 11 ist als Schlitten ausgeführt, der in Längsrichtung der Grundplatte 50 verschoben werden kann. Zu diesem Zweck sind in der Grundplatte 50 zwei in Längsrichtung verlaufende Führungen 52 ausgenommen, in die Vorsprünge des Schlittens eingreifen. Durch diese Anordnung kann eine stufenlose Längsverschiebung des Schlittens 11 erreicht werden. Die Translationsstellung des Schlittens 11 kann in mehreren Stufen fixiert werden. Vorliegend ist am Schlitten 11 ein vorgespannter Stift 51 angeordnet, der wahlweise in eines von mehreren in Längsrichtung der Grundplatte 50 beabstandeten Löchern eingreift. Selbstverständlich sind auch andere Mittel denkbar, mit denen eine stufenlose oder stufige Arretierung der Translationsstellung erreicht werden kann, wie beispielsweise eine Klemmbremse oder dergleichen.

Der Schlitten ist seinserseits aus zwei zueinander verschwenkbaren Stücken angeordnet, nämlich einem Grundstück 13 und einem Zwischenstück 14. Das Grundstück 13 ist anhand der Führungen in Längsrichtung der Grundplatte 50 verschiebbar gelagert und das Zwischenstück 14 ist verschwenkbar an dem Grundstück 13 gelagert. Das Verschwenken erfolgt anhand eines Schwenklagers 15, das im gezeigten Ausführungsbeispiel in Form zweier Bolzen ausgebildet ist, die in Bohrungen des Grundstücks 13 und des Zwischenstücks 14 gelagert sind. Selbstverständlich sind auch andere Lösungen denkbar. Die Schwenkachse verläuft in Querrichtung der Vorrichtung. Die Schwenkstellung des Zwischenstücks 14 kann in mehreren Stufen fixiert werden. Beispielsweise kann am Grundstück 13 ein vorgespannter Stift 16 angeordnet sein, der wahlweise in eines von mehreren beabstandeten Löchern am Zwischenstück 14 eingreift. Selbstverständlich sind auch andere Mittel denkbar, mit denen eine stufenlose oder stufige Arretierung der Schwenkstellung erreicht werden kann. Am Zwischenstück 14 ist ein Griff 17 vorgesehen, um eine einfachere Bedienung durch den Benutzer zu ermöglichen.

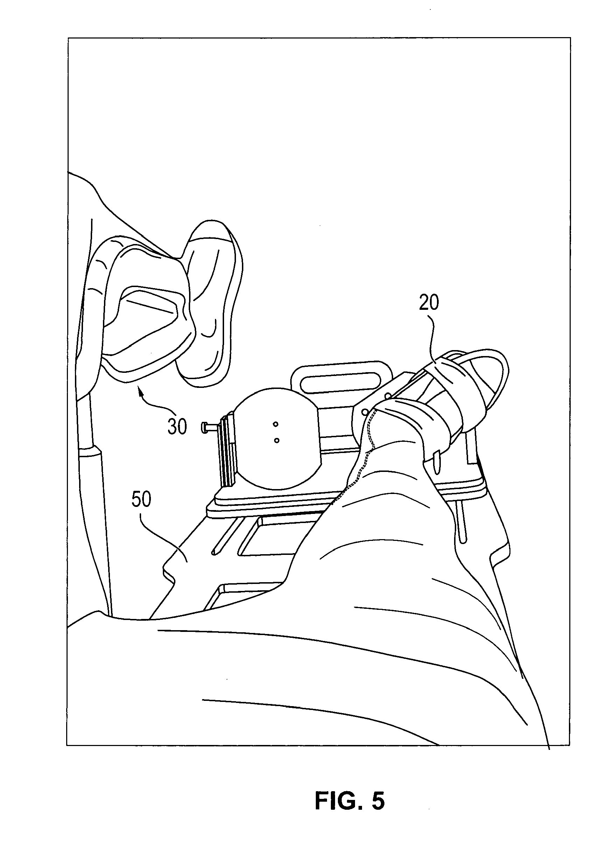

In Figur 5 wird die eben beschriebene Ausführungsform der erfindungsgemäßen Vorrichtung im Betriebszustand gezeigt, wobei die Beine eines Patienten in der Vorrichtung gelagert und fixiert sind.

Das rechte Bein des Patienten liegt flach auf der Grundplatte 50 auf und der rechte Fuß ist in der Bindung 20 fixiert. Die Bindung 20 ist um etwa 45° nach außen gedreht, sodass der Fuß und das Bein in einer nach außen ausgedrehten Stellung fixiert werden. Das Bein ist ansonsten im Wesentlichen gestreckt. Das linke Bein des Patienten wird von einem an der linken Seite der Grundplatte 50 angebrachten Beinteil 30 angehoben und befindet sich im abgewinkelten Zustand oberhalb der Grundplatte. Der linke Fuß ist nicht am Fußteil 10 fixiert und hängt frei.

Die gezeigte Beinstellung ermöglicht beispielsweise eine Bestrahlung der Innenseite des rechten Beines, wobei durch die Drehung des Beines auch ein Bestrahlungswinkel abgedeckt wird, der den Knochen leicht von hinten erfasst. Durch die Verwendung der Snowboardbindung mit den einfach zu bedienenden Ratschenverschlüssen ist der Fuß einfach in dem Fußteil fixierbar. Durch die einfach zu handhabenden Einstellmöglichkeiten hinsichtlich Rotationsstellung, Translationsstellung und Kippstellung des Beinteils kann ferner auf einfache Weise die gewünschte Beinstellung in reproduzierbarer Weise erreicht werden. Das andere Bein kann durch das Beinteil von der Grundplatte 50 abgehoben werden, um einen Bestrahlungsweg von innen (d.h. in der Abbildung von links) freizugeben.

In Figur 5 wird selbstverständlich nur eine von zahllosen Möglichkeiten gezeigt, die unteren Extremitäten eines Patienten zu fixieren.

## Patentansprüche

1. Vorrichtung zur Lagerung der unteren Extremitäten eines Patienten während der radiologischen Diagnostik bzw. Behandlung, wobei die Vorrichtung wenigstens ein Fußteil aufweist,
**dadurch gekennzeichnet,**
**dass** das Fußteil wenigstens eine rotierbar gelagerte Halterung für einen einzelnen Fuß und/oder den Unterschenkel des Patienten aufweist.

2. Vorrichtung zur Lagerung der unteren Extremitäten eines Patienten während der radiologischen Diagnostik bzw. Behandlung, vorzugsweise Vorrichtung nach Anspruch 1, wobei die Vorrichtung wenigstens ein Fußteil aufweist, **dadurch gekennzeichnet,**
**dass** das Fußteil eine Halterung für einen einzelnen Fuß und/oder den Unterschenkel des Patienten aufweist, wobei die Halterung Mittel zur festen Fixierung bzw. Immobilisierung des einzelnen Fußes und/oder Unterschenkels des Patienten aufweist.

3. Vorrichtung nach einem der vorhergehenden Ansprüche, **dadurch gekennzeichnet, dass** die Halterung eine Fußauflage und eine Befestigungsvorrichtung für den Fuß aufweist, wobei die Befestigungsvorrichtung vorzugsweise einen Schaft für den Unterschenkel und/oder ein Mittel zur Verhinderung eines Abhebens des Fußes umfasst.

4. Vorrichtung nach einem der vorhergehenden Ansprüche, **dadurch gekennzeichnet, dass** das Fußteil eine Basis aufweist und die Halterung vorzugsweise rotierbar an der Basis gelagert ist, wobei es sich bei der Basis vorzugsweise um einen im Wesentlichen in Längsrichtung der Vorrichtung verschiebbaren Schlitten handelt.

5. Vorrichtung nach einem der vorhergehenden Ansprüche, **dadurch gekennzeichnet, dass** die Halterung um eine im Wesentlichen in Querrichtung der Vorrichtung verlaufende Achse schwenkbar an der Vorrichtung und vorzugsweise an der Basis gelagert ist.

6. Vorrichtung nach einem der vorhergehenden Ansprüche, **dadurch gekennzeichnet, dass** die Halterung abnehmbar am Fußteil gelagert ist und/oder dass das Fußteil zwei unabhängig voneinander rotierbar an der Basis gelagerte Halterungen aufweist, die vorzugsweise baugleich und/oder spiegelverkehrt zueinander sind.

7. Vorrichtung nach einem der vorhergehenden Ansprüche, **dadurch gekennzeichnet, dass** die Vorrichtung ferner ein Beinteil mit einer Beinauflage für ein einzelnes Bein aufweist, die auf einem höhenverstellbaren Träger gelagert ist.

8. Vorrichtung nach einem der vorhergehenden Ansprüche, **dadurch gekennzeichnet, dass** die Vorrichtung eine Grundplatte aufweist, auf der das Fußteil und/oder das Beinteil auf der Grundplatte angeordnet sind.

9. Vorrichtung nach Anspruch 7 oder 8, **dadurch gekennzeichnet, dass** es sich bei dem Träger um einen Mast handelt, der vorzugsweise teleskopartig aus-und einfahrbar ist.

10. Vorrichtung nach einem der Ansprüche 7 bis 9, **dadurch gekennzeichnet, dass** das Beinteil abnehmbar an der Vorrichtung gelagert ist.

11. Vorrichtung nach einem der vorhergehenden Ansprüche, **dadurch gekennzeichnet**, die Vorrichtung eine Arretiervorrichtung und vorzugsweise eine Rastvorrichtung aufweist, die so ausgebildet ist, dass die Rotationsstellung der Halterung stufenlos oder in Stufen arretierbar ist.
